(19) Europäisches Patentamt
European Patent Office
Office européen des brevets

(11) Publication number: 0 306 904 B1

(12) # EUROPEAN PATENT SPECIFICATION

(45) Date of publication of patent specification: **28.07.93**

(51) Int. Cl.$^5$: **C07F 9/12**, C09K 15/32

(21) Application number: **88114555.1**

(22) Date of filing: **07.09.88**

The file contains technical information submitted after the application was filed and not included in this specification

(54) **Phosphoric acid diesters and salts thereof.**

(30) Priority: **11.09.87 JP 228845/87**

(43) Date of publication of application:
**15.03.89 Bulletin 89/11**

(45) Publication of the grant of the patent:
**28.07.93 Bulletin 93/30**

(84) Designated Contracting States:
**AT BE CH DE ES FR GB GR IT LI LU NL SE**

(56) References cited:
**EP-A- 0 127 471**
**US-A- 3 749 680**

**CHEMICAL ABSTRACTS, vol. 111, no. 6, 07 August 1989, Columbus, OH (US); p. 387, no. 45280t&NUM;**

(73) Proprietor: **Senju Pharmaceutical Co., Ltd.**
**6-1, Hiranomachi 3-chome Higashi-ku**
**Osaka(JP)**

(72) Inventor: **Yoshida, Koichi**
**1652-1, Sakazu**
**Kurashiki-shi Okayama(JP)**
Inventor: **Takigawa, Tetsuo**
**160-4, Bakuro-cho**
**Kurashiki-shi Okayama(JP)**
Inventor: **Ogata, Kazumi**
**B-701, 8, Kamishinden 4-chome**
**Toyonaka-shi Osaka(JP)**
Inventor: **Yoshida, Kenichi**
**2, Kitahonmachi 2-chome**
**Itami-shi Hyogo(JP)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3. 10/3.6/3.3. 1)

## Description

1. Field of the Invention

The present invention relates to novel phosphoric acid diesters and salts thereof having anti-oxidative activity and the like and processes for production thereof.

2. Description of Prior Art

US-A-3 749 680 discloses an antioxidant composition consisting essentially of isoascorbic acid-3-phosphate, isoascorbic acid-2-phosphate, isoascorbic acid-2,3-diphosphate and isoascorbic acid-2,3-cyclic phosphate and the alkali metal salts thereof.

Hindered phenols, e.g. 3,5-di-tert-butyl-4-hydroxytoluene (BHT) and octadecyl 3-(3,5-di-tert-butyl-4-hydroxyphenyl)propionate (Irganox 1076), hindered amines, e.g. 6-ethoxy-1,2-dihydro-2,2,4-trimethylquinoline (ethoxyquin), sulfides and phosphides are utilized as antioxidants for plastics and as anti-aging agents for rubbers. On the other hand, for the purpose of preventing oxidation of processed foodstuffs, propyl gallate and BHT are utilized in Japan, and vitamin E, though only in a limited field, is also used in Europe and the United States along with those materials.

One of the inventors of the present invention has already reported that a phosphoric acid diester of L-ascorbic acid and DL-$\alpha$ -tocopherol and its salts may be useful as prophylactics and therapeutics for cataract and climacteric disturbances, as antiinflammatory agents and also as materials for cosmetics for skin care (Japanese Unexamined Patent Publication (KOKAI) Nos. 59-219295 and 62-145019.)

While demands for processed foods have been rapidly expanding, it has been reported that lipidperoxide, which occurs in processed foodstuffs during storage, may exert harmful influences on human body. Development of highly safe stabilizers of processed foodstuffs has been thereby desired.

Having been noted as a highly safe antioxidant, vitamin E is used in Europe and the United States in certain field of processed food. It however has not come to be a widely used agent because it has only a low solubility in water and it readily acquire a color by oxidation.

The present invention is directed to:

(1) The novel phosphoric acid diesters represented by the following formula (I):

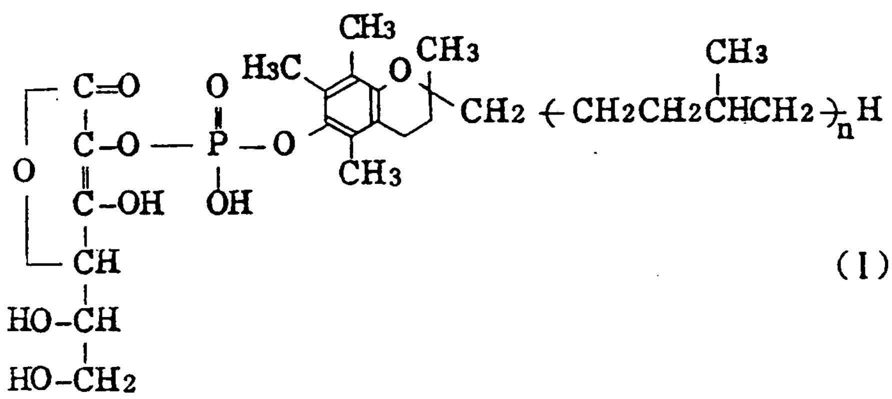

(I)

(wherein n is an integer selected from the group consisting of 0,1, 2, 4 and 5) and salts thereof, and

(2) The process for producing the compounds mentioned hereinabove, which comprises the following sequence of; treatment of a chroman derivative seleced from the group having the formula:

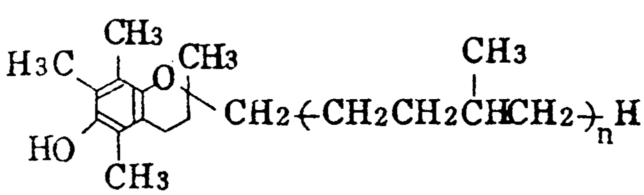

(II)

with a halophosphorylating agent, then subjecting the resulting chromanyl phosphodihalide to the

reaction with an ascorbic acid whose $C_5$- and $C_6$-hydroxy groups are protected with a protecting group or protecting groups, and elimination of the protecting group(s) from the reaction product.

The purpose of the present invention is to provide phosphoric acid diesters and their salts which are highly safe and effective, water-soluble antioxidants.

The problems mentioned above have been solved by providing phosphoric acid diesters .

The salts of the phosphoric acid diesters represented by the formula (I) include a salt with an alkaline metal such as sodium or potassium, a salt with an alkali earth metal such as magnesium or calcium, an ammonium salt and an salt with tert-ammonium such as choline.

The phosphoric acid diesters represented by the following formula (I):

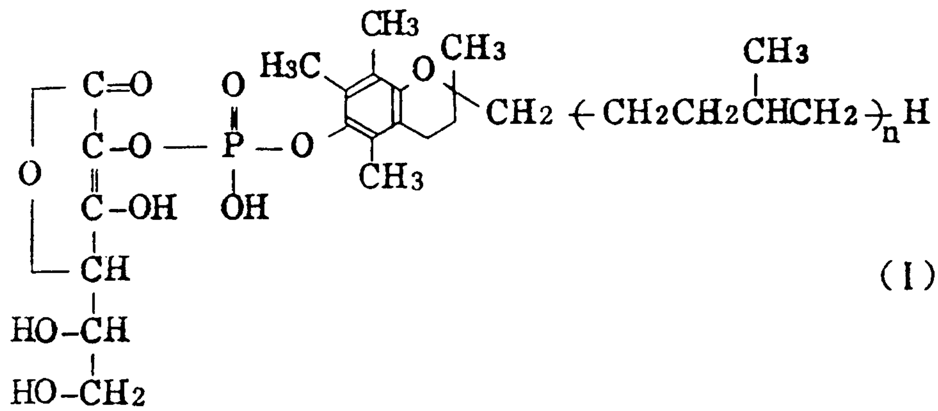

(I)

(wherein n is an integer selected from the group consisting of 0, 1, 2, 4 and 5) and their salts may be useful as antioxidants for the products mentioned below. The compounds are also anticipated to be utilized as anti-cataract therapeutics.

Since the phosphoric acid diesters represented by the formula (I) and the salts thereof are soluble in water and low in toxicity, they may be especially useful as antioxidants for processed foodstuffs and aqueous cosmetics such as toilet waters, hair tonics, Eau de Colonges, perfumes and the like. The phosphoric acid diesters represented by the formula (I) and the salts thereof may give neither peculiar tastes nor peculiar smells to processed foodstuffs in which they are employed. The amount of a phosphoric acid diester illustrated generally by the formula (I) or a salt thereof, when it is used as an antioxidant, may be selected from the range of approx. 0.001 to 20 % depending on the stability of the product in which it is employed.

Additionally, the phosphoric acid diesters represented by the formula (I) and the salts thereof have platelet aggregation inhibiting activity, and their use as therapeutics for thrombosis and angina pectoris and the like are thereby anticipated.

The phosphoric acid diesters represented by the formula (I) may be prepared by, e.g. a similar method to the one described in Japanese Unexamined Patent Publication (KOKAI) No. 59-219295 This method is conducted by treating a chroman derivative illustrated generally by the formula (II):

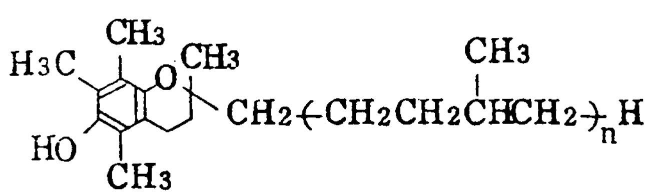

(II)

(wherein n is the same as defined hereinbefore,) with a halophosphorylating agent, then subjecting the reaction product, which is a chromanyl phosphorodihalide, to the reaction with an ascorbic acid each of whose $C_5$- and $C_6$-hydroxyl groups is protected with a protecting group, and finally eliminating the both protecting groups from the resulting product of the reaction. In more detail, a chroman derivative illustrated generally by the formula (II) is first treated with a halophosphorylating agent such as phosphorous oxychloride or phosphorous oxybromide. This reaction may be carried out in organic solvents such as

benzene, toluene or xylene, in the presence of an basic acid acceptor such as pyridine or triethylamine, under a temperature from ice-cold to ambient temperature. The chromanyl phosphorodihalide thus obtained is then rendered to the reaction with an ascorbic acid each of whose $C_5$- and $C_6$-hydroxyl groups is protected. For the protection of the $C_5$- and $C_6$-hydroxyl groups of ascorbic acid, acyl group such as acetyl group and, preferably, isopropylidene group may be employed. The reaction may be conducted in organic solvents such as tetrahydrofran in the presence of a basic acid acceptor such as pyridine, at a temperature from ice-cold to ambient temperature. The protecting groups are then eliminated from the resulting product of the reaction. This hydrolysis reaction may be conducted under a mild condition employing dilute inorganic or organic acid, e.g. 0.5-1 N hydrochloric acid, 0.5-1 N sulfuric acid, 0.5-1 N phosphoric acid or 0.5-1 N trichloroacetic acid. A corresponding halophosphoric acid (ascorbic acid-2-O)(chromanyl-6) ester, when contained in the reaction product mixture, may be transformed into the phosphoric acid diester illustrated generally by the formula (I) in this hydrolysis reaction. Thus, a phosphoric acid diester illustrated generally by the formula (I) may be prepared.

A salt of a phosphoric acid diester illustrated generally by the formula (I) may be prepared with ease from the phosphoric acid diester illustrated generally by the formula (I) and a proper base.

The following experiments are presented as an example to demonstrate the anti-oxidative activity of the phosphoric acid diesters represented by the formula (I) and salts thereof.

**Experiments**

Anti-oxidative activity of the compounds prepared in example 1 and example 4 were determined using, as an index, oxidation of mixture micelles, which were prepared from phospholipid (egg lecithin) and ethanol, by ferrous-ion/ascorbic acid

To a solution of approx. 78 mg of egg lecithin in 2 ml of ethanol was added gradually 5 mM HEPES buffer solution (pH 7.2), under sonication (50 W) and ice-cooling, to make a 100-ml suspension. To 1,000 $\mu$ l of the suspension were added 200 $\mu$ l of 2.6 x $10^{-4}$ M solution of one of the test compounds in 50 v/v % ethanol, 50 $\mu$ l of 5.0 x $10^{-5}$ M sodium ascorbate solution in water and 50 $\mu$ l of 2.5 x $10^{-6}$ M ferrous sulfate solution in water. The mixture was placed in a water bath at 25 °C for 15 min for oxidation. Instantly after the reaction, 50 $\mu$ l of 0.1 % hydroquinone solution in ethanol was added to stop the reaction. To the mixture were added 200 $\mu$ l of 20 w/v % trichloroacetic acid and 2,000 $\mu$ l of 50 v/v % acetic acid containing thiobarbituric acid (Merck) at the concentration of 0.35 %, followed by heating for 15 min under reflux at 100 °C. After cooling, absorption within the range of 450-600 nm was measured by spectrophotography, within which range increase in absorbance at approx. 530-540 nm was determined. Rates of inhibition were calculated from the difference in absorbance between the values thus obtained and those from controls which were prepared by the same method except for the addition of the test compound.

The results were as follows.

| Tested compound | Final concentration | |
|---|---|---|
| | 4.0 x $10^{-5}$ | 4.0 x $10^{-6}$ |
| | Rate of inhibition (%) | |
| The compound prepared in example 1 | 94.44 | 89.57 |
| The compound prepared in example 4 | 99.90 | 95.22 |

As shown above, the both compounds prepared in example 1 and example 4 exhibited nearly complete inhibition of oxidation.

Those compounds prepared in example 1 and example 4 were further evaluated for their anti-oxidative activity in a model employing UV-induced oxidation of the lens, the both exhibiting a significant inhibition of accumulation of malonedialdehyde.

As demonstrated by the results of the above experiments, phosphoric acid diesters represented by the formula (I) and salts thereof, particularly the compound (I) wherein n is 4 and salts thereof, have excellent antioxidative activity.

**Examples**

The following examples are presented as a further illustration of the present invention.

**Example 1:**

<u>The preparation of potassium salt of phosphoric acid (L-ascorbic acid-2-O)(2,5,7,8-tetramethyl-2-(4,8-dimethylnonyl)chromanyl-6) ester</u>

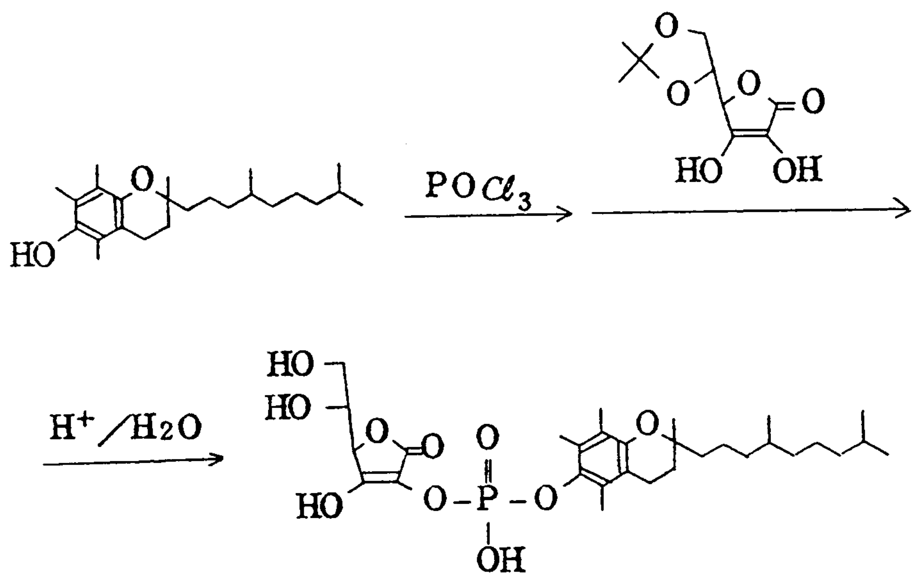


9.16 g of phosphorous oxychloride was dissolved in a mixture solution of 15 ml of pyridine and 45 ml of benzene. To the solution thus obtained, a mixed solution of 5.01 g (14 mmol) of 2,5,7,8-tetramethyl-2-(4,8-dimethylnonyl)-6-chromanol and 2.3 g of pyridine in 40 ml of benzene was added dropwise under stirring and ice-cooling. After that, the mixture was stirred for three hours. Following the removal of precipitated pyridine hydrochloride by decantation, the solution was evaporated to dryness under reduced pressure. To the oily residue thus obtained was then added 20 ml of benzene to prepare a benzene solution.

Separately, 6.14 g (28 mmol) of 5,6-isopropylidene ascorbic acid, which was prepared by acetonization of L-ascorbic acid, and 5 ml of pyridine were dissolved in 140 ml of tetrahydrofran. To the solution thus obtained was added dropwise the above prepared benzene solution under stirring and ice-cooling. After that, the mixture was stirred for approx. further one hour, then the reaction mixture was filtered through a small amount of silica-gel. From the filtrate, the solvent was evaporated off under reduced pressure. The oily residue thus obtained was dissolved in 20 ml of ethanol, followed by the addition of 80 ml of 0.5 N hydrochloric acid and stirring at 60 °C for approx. 30 min. After cooling, the reaction mixture was extracted with ethyl acetate. The extract, after dried over anhydrous magnesium sulfate, was evaporated to remove the solvent. Thus, phosphoric acid (L-ascorbic acid-2-O)(2,5,7,8-tetramethyl-2-(4,8-dimethyl-nonyl)-chromanyl-6) ester was obtained as crude product. The crude product was then dissolved in 15 ml of methanol, and to the solution, a methanol solution of potassium hydroxide was added dropwise until the pH of the resulting solution reached 7.0. Faintly brownish white crystals subsequently precipitated. The crystals were washed with methanol and then with acetone, and recrystallized from water-acetone-ethanol to yield 4.40 g of potassium salt of phosphoric acid (L-ascorbic acid-2-O)(2,5,7,8-tetramethyl-2-(4,8-dimethylnonyl)-chromanyl-6) ester with the following physicochemical properties as white powder.

UV-absorption spectrum: 259 nm (in water)

| Elemental analysis; for $C_{30}H_{45}O_{10}PK_2.H_2O$ : | | |
|---|---|---|
| Found : | C, 51.53 % | H, 7.02 % |
| Calcd.: | C, 51.87 % | H, 6.82 % |

IR-absorption spectrum (KBr disc) :
2940, 2860, 1737, 1590, 1464,
1415, 1246, 1086 $cm^{-1}$

NMR spectrum (90 MHz) $\delta$ HMS/$D_2O$ :

4.44(1H), 3.93(1H), 3.69(2H)(ascorbic acid unit);
0.83(9H), 1.0-1.6(17H), 1.70(2H),
2.07(9H), 2.53(2H)

**Example 2:**

The preparation of potassium salt of phosphoric acid (L-ascorbic acid-2-O)(2,2,5,7,8-pentamethylchromanyl-6) ester

The same reaction and purification procedures as in example 1 were followed except that 3.08 g (14 mmol) of 2,2,5,7,8-pentamethyl-6-chromanol was used in this example instead of 5.01 g (14 mmol) of 2,5,7,8-tetramethyl-2-(4,8-dimethylnonyl)-6-chromanol used in example 1. 2.3 g of potassium salt of phosphoric acid (L-ascorbic acid-2-O)(2,2,5,7,8-pentamethyl-chromanyl-6) ester with the following physicochemical properties was thus obtained as white powder.

UV-absorption spectrum: 265 nm (in water)

| Elemental analysis; for $C_{20}H_{25}O_{10}PK_2.2H_2O$ : | | |
|---|---|---|
| Found : | C, 41.73 % | H, 5.43 % |
| Calcd.: | C, 42.11 % | H, 5.12 % |

IR-absorption spectrum (KBr disc) :
-3150, 1740, 1600(weak), 1460,
1420, 1240, 1080 $cm^{-1}$
NMR spectrum (90 MHz) $\delta$ HMS/$D_2O$ :
4.48(1H), 3.98(1H), 3.70(2H)(ascorbic acid unit);
1.25(6H), 1.72(2H), 2.07(6H),
2.10(3H), 2.57(2H)

**Example 3:**

The preparation of potassium salt of phosphoric acid (L-ascorbic acid-2-O)(2,5,7,8-tetramethyl-2-(4-methylpentyl)chromanyl-6) ester.

The same reaction and purification procedures as in example 1 were followed except that 4.07 g (14 mmol) of 2,5,7,8-tetramethyl-2-(4-methylpentyl)-6-chromanol was used in this example instead of 5.01 g (14 mmol) of 2,5,7,8-tetramethyl-2-(4,8-dimethylnonyl)-6-chromanol used in example 1. 3.8 g of potassium salt of phosphoric acid (L-ascorbic acid-2-O) (2,5,7,8-tetramethyl-2-(4-methylpentyl)chromanyl-6) ester with the following physicochemical properties was thus obtained as faintly brownish white powder.

UV-absorption spectrum: 261 nm

| Elemental analysis; for $C_{25}H_{35}O_{10}PK_2.2H_2O$ : | | |
|---|---|---|
| Found : | C, 46.47 % | H, 6.42 % |
| Calcd.: | C, 46.88 % | H, 6.14 % |

IR-absorption spectrum (KBr disc) :
2950, 2850, 1740, 1610, 1460,
1420, 1250, 1090 $cm^{-1}$
NMR spectrum (90 MHz) $\delta$ HMS/$D_2O$ :
4.51(1H), 4.02(1H), 3.73(2H)(ascorbic acid unit);
0.83(6H), 1.0-1.6(10H), 1.71(2H),
2.05(9H), 2.55(2H)

**Example 4:**

The preparation of potassium salt of phosphoric acid (L-ascorbic acid-2-O)(2,5,7,8-tetramethyl-2-(4,8,12,16-tetramethylheptadecyl)chromanyl-6) ester.

The same reaction and purification procedures as in example 1 were followed except that 7.01 g (14 mmol) of 2,5,7,8-tetramethyl-2-(4,8,12,16-tetramethylheptadecyl)-6-chromanol was used in this example instead of 5.01 g (14 mmol) of 2,5,7,8-tetramethyl-2-(4,8-dimethylnonyl)-6-chromanol used in example 1. 5.3 g of potassium salt of phosphoric acid (L-ascorbic acid-2-O)(2,5,7,8-tetramethyl-2-(4,8,12,16-tetramethylheptadecyl)-chromanyl-6) ester with the following physicochemical properties was thus obtained as faintly brownish white powder.

UV-absorption spectrum: 253 nm

| Elemental analysis; for $C_{40}H_{65}O_{10}PK_2.H_2O$ : | | |
|---|---|---|
| Found :<br>Calcd.: | C, 57.91 %<br>C, 57.68 % | H, 8.46 %<br>H, 8.11 % |

IR-absorption spectrum (KBr disc) :
2950, 2860, 1730, 1600, 1467,
1417, 1250, 1090 $cm^{-1}$

**Example 5:**

The preparation of potassium salt of phosphoric acid (L-ascorbic acid-2-O)(2,5,7,8-tetramethyl-2-(4,8,12,16,20-pentamethylheneicosyl)chromanyl-6) ester.

The same reaction and purification procedures as in example 1 were followed except that 7.99 g (14 mmol) of 2,5,7,8-tetramethyl-2-(4,8,12,16,20-pentamethylheneicosyl)-6-chromanol was used in this example instead of 5.01 g (14 mmol) of 2,5,7,8-tetramethyl-2-(4,8-dimethylnonyl)-6-chromanol used in example 1. 5.7 g of potassium salt of phosphoric acid (L-ascorbic acid-2-O)(2,5,7,8-tetramethyl-2-(4,8,12,16,20-pentamethylheneicosyl)chromanyl-6) ester with the following physicochemical properties was thus obtained as brown powder.

UV-absorption spectrum: 250 nm

| Elemental analysis; for $C_{45}H_{75}O_{10}PK_2$ : | | |
|---|---|---|
| Found :<br>Calcd.: | C, 60.71 %<br>C, 61.07 % | H, 8.90 %<br>H, 8.54 % |

IR-absorption spectrum (KBr disc) :
2950, 2850, 1735, 1610, 1455,
1420, 1255, 1080 $cm^{-1}$

## Claims

**Claims for the following Contracting States : AT, BE, CH, DE, FR, GB, GR, IT, LI, LU, NL, SE**

1. A compound selected from the group having the formula:

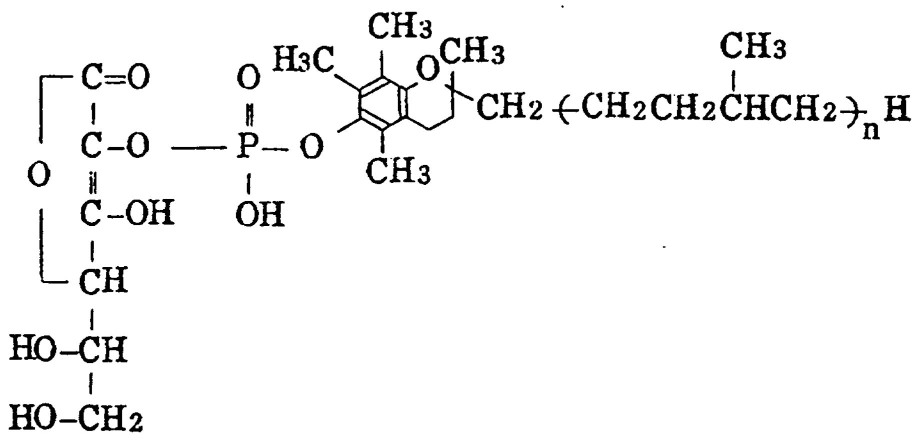

wherein n is an integer selected from the group consisting of 0, 1, 2, 4 and 5, and a salt thereof.

2. The compound of claim 1 which is phosphoric acid (L-ascorbic acid-2-O)(2,5,7,8-tetramethyl-2-(4,8-dimethylnonyl)chromanyl-6) ester and a salt thereof.

3. The compound of claim 1 which is phosphoric acid (L-ascorbic acid-2-O)(2,2,5,7,8-pentamethyl-chromanyl-6) ester and a salt thereof.

4. The compound of claim 1 which is phosphoric acid (L-ascorbic acid-2-O) (2,5,7,8-tetramethyl-2-(4-methylpentyl)chromanyl-6) ester and a salt thereof.

5. The compound of claim 1 which is phosphoric acid (L-ascorbic acid-2-O)(2,5,7,8-tetramethyl-2-(4,8,12,16-tetramethylheptadecyl)-chromanyl-6) ester and a salt thereof.

6. The compound of claim 1 which is phosphoric acid (L-ascorbic acid-2-O)(2,5,7,8-tetramethyl-2-(4,8,12,16,20-pentamethylheneicosyl)chromanyl-6) ester and a salt thereof.

7. A process of producing a compound as claimed in claims 1-6, which comprises the following sequence of; treatment of a chroman derivative selected from the group having the formula:

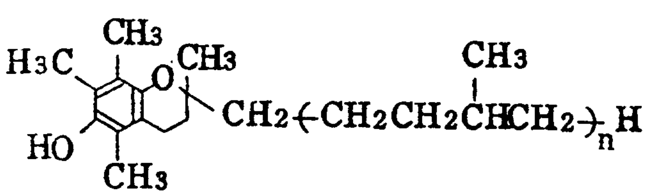

with a halophosphorylating agent, then subjecting the resulting chromanyl phosphodihalide to the reaction with an ascorbic acid whose $C_5$- and $C_6$-hydroxy groups are protected with a protecting group or protecting groups, and elimination of the protecting group(s) from the reaction product.

8. The process of claim 7 wherein the halophosphorylating agent is phosphorous oxychloride or phosphorous oxybromide.

**9.** The process of claim 7 wherein the treatment of the chroman derivative with the halophosphorylating agent is conducted in benzene, toluene or xylene in the presence of pyridine or triethylamine.

**10.** The process of claim 7 wherein the protecting group is isopropylidene or acetyl.

**11.** The process of claim 7 wherein the reaction of chromanyl phosphodihalide with an ascorbic acid whose $C_5$- and $C_6$-hydroxy groups are protected with a protecting group or protecting groups is conducted in tetrahydrofran in the presence of pyridine or triethylamine.

**12.** The process of claim 7 wherein the elimination of the protecting group(s) is conducted in acidic condition employing hydrochloric acid, sulfuric acid or phosphoric acid.

**13.** Use of the compounds according to claims 1 to 6 or obtained by the process of claims 7 to 12 as an antioxidant.

**14.** A composition comprising a compound as claimed in claims 1 to 6 or obtained by the process of claims 7 to 12 as an antioxidant.

**15.** The composition of claim 14 for use in processed foodstuffs and aqueous cosmetics.

**Claims for the following Contracting State : ES**

**1.** A process of producing a compound selected from the group having the formula:

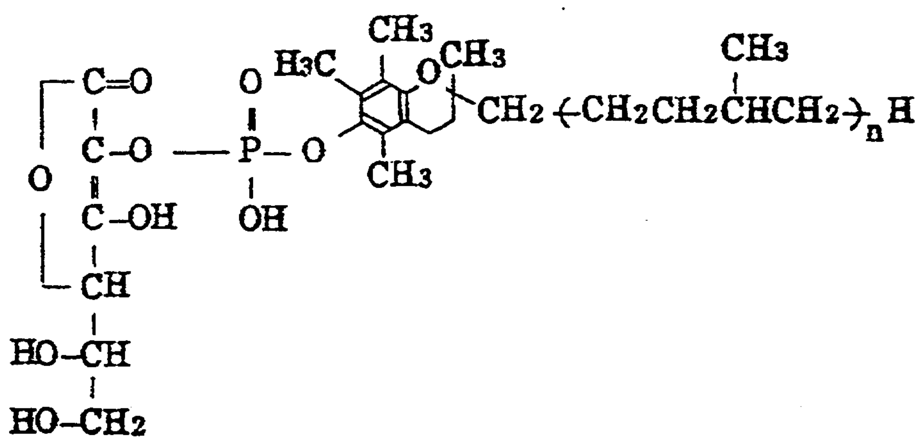

wherein n is an integer selected from the group consisting of 0, 1, 2, 4 and 5, and salts thereof, which comprises the following sequence of; treatment of a chroman derivative selected from the group having the formula:

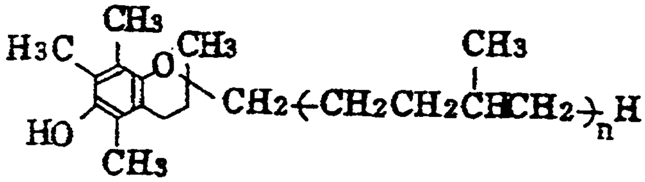

with a halophosphorylating agent, then subjecting the resulting chromanyl phosphodihalide to the reaction with an ascorbic acid whose $C_5$- and $C_6$-hydroxy groups are protected with a protecting group or protecting groups, and elimination of the protecting group(s) from the reaction product.

**2.** The process of claim 1 wherein the final product is phosphoric acid (L-ascorbic acid-2-O)(2,5,7,8-tetramethyl-2-(4,8-dimethylnonyl)chromanyl-6) ester and a salt thereof.

3. The process of claim 1 wherein the final product is phosphoric acid (L-ascorbic acid-2-O)(2,2,5,7,8-pentamethyl-chromanyl-6) ester and a salt thereof.

4. The process of claim 1 wherein the final product is phosphoric acid (L-ascorbic acid-2-O) (2,5,7,8-tetramethyl-2-(4-methylpentyl)chromanyl-6) ester and a salt thereof.

5. The process of claim 1 wherein the final product is phosphoric acid (L-ascorbic acid-2-O)(2,5,7,8-tetramethyl-2-(4,8,12,16-tetramethylheptadecyl)-chromanyl-6) ester and a salt thereof.

6. The process of claim 1 wherein the final product is phosphoric acid (L-ascorbic acid-2-O)(2,5,7,8-tetramethyl-2-(4,8,12,16,20-pentamethylheneicosyl)chromanyl-6) ester and a salt thereof.

7. The process of claim 1 wherein the halophosphorylating agent is phosphorous oxychloride or phosphorous oxybromide.

8. The process of claim 1 wherein the treatment of the chroman derivative with the halophosphorylating agent is conducted in benzene, toluene or xylene in the presence of pyridine or triethylamine.

9. The process of claim 1 wherein the protecting group is isopropylidene or acetyl.

10. The process of claim 1 wherein the reaction of chromanyl phosphodihalide with an ascorbic acid whose $C_5$- and $C_6$-hydroxy groups are protected with a protecting group or protecting groups is conducted in tetrahydrofran in the presence of pyridine or triethylamine.

11. The process of claim 1 wherein the elimination of the protecting group(s) is conducted in acidic condition employing hydrochloric acid, sulfuric acid or phosphoric acid.

12. A composition comprising a compound obtained by the process of claims 1 to 11 as an antioxidant.

13. A processed foodstuff comprising a compound obtained by claims 1 to 11.

14. An aqueous cosmetic comprising a compound obtained by claims 1 to 11.

**Patentansprüche**

**Patentansprüche für folgende Vertragsstaaten : AT, BE, CH, DE, FR, GB, GR, IT, LI, LU, NL, SE**

1. Verbindung, ausgewählt aus der Gruppe mit der Formel

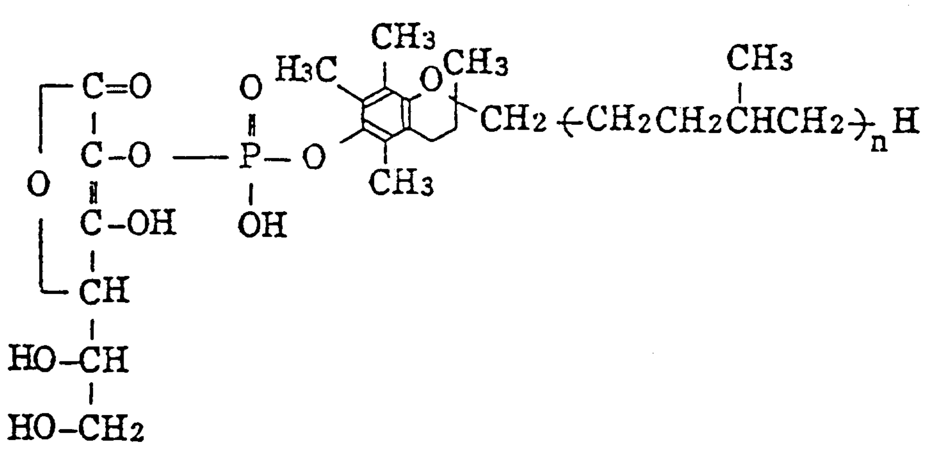

in der n eine aus der aus 0, 1, 2, 4 und 5 bestehenden Gruppe ausgewählte ganze Zahl ist, und ein Salz derselben.

2. Verbindung nach Anspruch 1, die Phosphorsäure-(L-ascorbinsäure-2-O)-(2,5,7,8-tetramethyl-2-(4,8-dimethylnonyl)chromanyl-6)-ester ist, und ein Salz derselben.

3. Verbindung nach Anspruch 1, die Phosphorsäure-(L-ascorbinsäure-2-O)-(2,2,5,7,8-pentamethylchromanyl-6)-ester ist, und ein Salz derselben.

4. Verbindung nach Anspruch 1, die Phosphorsäure-(L-ascorbinsäure-2-O)-(2,5,7,8-tetramethyl-2-(4-methylpentyl)-chromanyl-6)-ester ist, und ein Salz derselben.

5. Verbindung nach Anspruch 1, die Phosphorsäure-(L-ascorbinsäure-2-O)-(2,5,7,8-tetramethyl-2-(4,8,12,16-tetramethylheptadecyl)chromanyl-6)-ester ist, und ein Salz derselben.

6. Verbindung nach Anspruch 1, die Phosphorsäure-(L-ascorbinsäure-2-O)-(2,5,7,8-tetramethyl-2-(4,8,12,16,20-pentamethylheneicosyl)chromanyl-6)-ester ist, und ein Salz derselben.

7. Verfahren zur Herstellung einer Verbindung, wie sie in den Ansprüchen 1 bis 6 beansprucht wird, umfassend die nachfolgende Sequenz
der Behandlung eines Chroman-Derivats, das aus der Gruppe mit der Formel

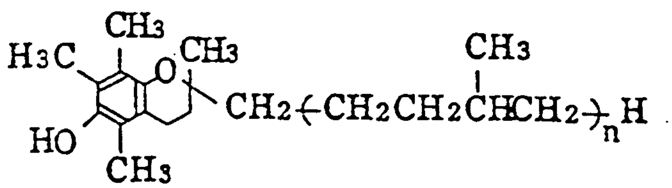

ausgewählt ist, mit einem Halogenphosphorylierungsmittel,
der anschließenden Umsetzung des resultierenden Chromanylphosphodihalogenids mit einer Ascorbinsäure, deren $C_5$- und $C_6$-Hydroxy-Gruppen mit einer Schutzgruppe oder mit Schutzgruppen geschützt sind, und
der Eliminierung der Schutzgruppe(n) aus dem Reaktionsprodukt.

8. Verfahren nach Anspruch 7, worin das Halogenphosphorylierungsmittel Phosphoroxidchlorid oder Phosphoroxidbromid ist.

9. Verfahren nach Anspruch 7, worin die Behandlung des Chroman-Derivats mit dem Halogenphosphorylierungsmittel in Benzol, Toluol oder Xylol in Gegenwart von Pyridin oder Triethylamin durchgeführt wird.

10. Verfahren nach Anspruch 7, worin die Schutzgruppe Isopropyliden oder Acetyl ist.

11. Verfahren nach Anspruch 7, worin die Umsetzung des Chromanylphosphodihalogenids mit einer Ascorbinsäure, deren $C_5$- und $C_6$-Hydroxy-Gruppen mit einer Schutzgruppe oder mit Schutzgruppen geschützt sind, in Tetrahydrofuran in Gegenwart von Pyridin oder Triethylamin durchgeführt wird.

12. Verfahren nach Anspruch 7, worin die Eliminierung der Schutzgruppe(n) unter sauren Bedingungen unter Einsatz von Salzsäure, Schwefelsäure oder Phosphorsäure durchgeführt wird.

13. Verwendung der Verbindungen nach den Ansprüchen 1 bis 6 oder der nach den Verfahren der Ansprüche 7 bis 12 erhaltenen Verbindungen als Antioxidationsmittel.

14. Zusammensetzung, umfassend eine Verbindung nach den Ansprüchen 1 bis 6 oder eine nach den Verfahren der Ansprüche 7 bis 12 erhaltene Verbindung als Antioxidationsmittel.

15. Zusammensetzung nach Anspruch 14 zur Verwendung in verarbeiteten Nahrungsmitteln und wäßrigen Schönheitspflegemitteln.

**Patentansprüche für folgenden Vertragsstaat : ES**

1. Verfahren zur Herstellung einer Verbindung, die aus der Gruppe mit der Formel

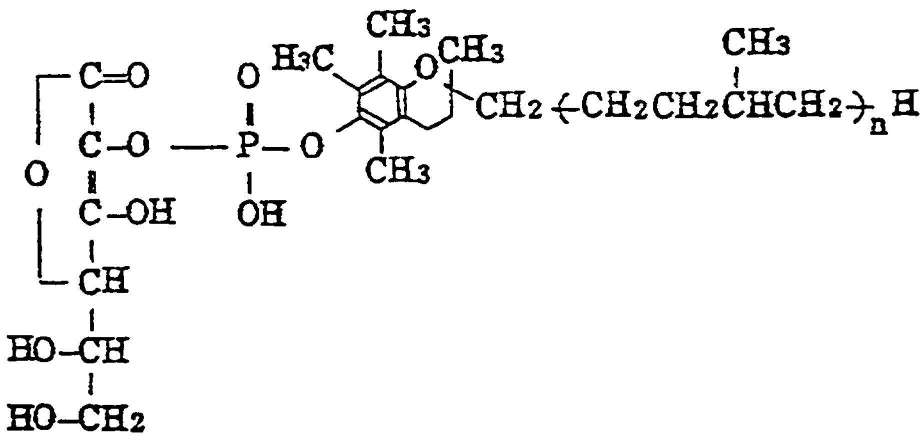

ausgewählt ist, in der n eine aus der aus 0, 1, 2, 4 und 5 bestehenden Gruppe ausgewählte ganze Zahl ist,
und von Salzen derselben,
umfassend die nachfolgende Sequenz
der Behandlung eines Chroman-Derivats, das aus der Gruppe mit der Formel

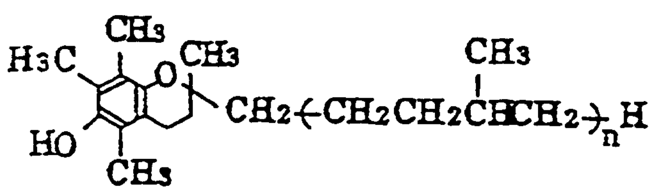

ausgewählt ist, mit einem Halogenphosphorylierungsmittel,
der anschließenden Umsetzung des resultierenden Chromanylphosphodihalogenids mit einer Ascorbinsäure, deren $C_5$- und $C_6$-Hydroxy-Gruppen mit einer Schutzgruppe oder mit Schutzgruppen geschützt sind, und
der Eliminierung der Schutzgruppe(n) aus dem Reaktionsprodukt.

2. Verfahren nach Anspruch 1, worin das Endprodukt Phosphorsäure-(L-ascorbinsäure-2-O)-(2,5,7,8-tetramethyl-2-(4,8-dimethylnonyl)chromanyl-6)-ester und ein Salz desselben ist.

3. Verfahren nach Anspruch 1, worin das Endprodukt Phosphorsäure-(L-ascorbinsäure-2-O)-(2,2,5,7,8-pentamethylchromanyl-6)-ester und ein Salz desselben ist.

4. Verfahren nach Anspruch 1, worin das Endprodukt Phosphorsäure-(L-ascorbinsäure-2-O)-(2,5,7,8-tetramethyl-2-(4-methylpentyl)chromanyl-6)-ester und ein Salz desselben ist.

5. Verfahren nach Anspruch 1, worin das Endprodukt Phosphorsäure-(L-ascorbinsäure-2-O)-(2,5,7,8-tetramethyl-2-(4,8,12,16-tetramethylheptadecyl)chromanyl-6)-ester und ein Salz desselben ist.

6. Verfahren nach Anspruch 1, worin das Endprodukt Phosphorsäure-(L-ascorbinsäure-2-O)-(2,5,7,8-tetramethyl-2-(4,8,12,16,20-pentamethylheneicosyl)chromanyl-6)-ester und ein Salz desselben ist.

7. Verfahren nach Anspruch 1, worin das Halogenphosphorylierungsmittel Phosphoroxidchlorid oder Phosphoroxidbromid ist.

8. Verfahren nach Anspruch 1, worin die Behandlung des Chroman-Derivats mit dem Halogenphosphorylierungsmittel in Benzol, Toluol oder Xylol in Gegenwart von Pyridin oder Triethylamin durchgeführt wird.

9. Verfahren nach Anspruch 1, worin die Schutzgruppe Isopropyliden oder Acetyl ist.

10. Verfahren nach Anspruch 1, worin die Umsetzung des Chromanylphosphodihalogenids mit einer Ascorbinsäure, deren $C_5$- und $C_6$-Hydroxy-Gruppen mit einer Schutzgruppe oder mit Schutzgruppen geschützt sind, in Tetrahydrofuran in Gegenwart von Pyridin oder Triethylamin durchgeführt wird.

11. Verfahren nach Anspruch 1, worin die Eliminierung der Schutzgruppe(n) unter sauren Bedingungen unter Einsatz von Salzsäure, Schwefelsäure oder Phosphorsäure durchgeführt wird.

12. Zusammensetzung, umfassend eine nach den Verfahren der Ansprüche 1 bis 11 erhaltene Verbindung als Antioxidationsmittel.

13. Verarbeitetes Nahrungsmittel, umfassend eine gemäß den Ansprüchen 1 bis 11 erhaltene Verbindung.

14. Wäßriges Schönheitspflegemittel, umfassend eine gemäß den Ansprüchen 1 bis 11 erhaltene Verbindung.

**Revendications**
**Revendications pour les Etats contractants suivants : AT, BE, CH, DE, FR, GB, GR, IT, LI, LU, NL, SE**

1. Composé choisi dans le groupe de formule

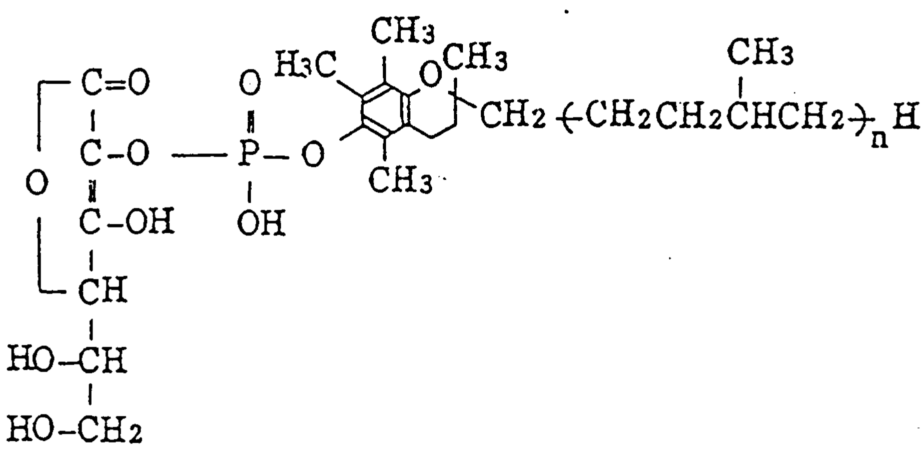

dans laquelle n est un nombre entier choisi dans le groupe constitué par 0, 1, 2, 4 et 5 et un de ses sels.

2. Composé de la revendication 1, qui est le (L-acide ascorbique-2-O)-2,5,7,8-tétraméthyl-2-(4,8-diméthylnonyl)chromanyl-6)ester de l'acide phosphorique et un de ses sels.

3. Composé de la revendication 1, qui est le (L-acide ascorbique-2-O)(2,2,5,7,8-pentaméthyl-chromanyl-6)-ester de l'acide phosphorique et un de ses sels.

4. Composé de la revendication 1 qui est le (L-acide ascorbique-2-O)(2,5,7,8-tétraméthyl-2-(4-méthylpentyl)chromanyl-6)ester de l'acide phosphorique.

5. Composé de la revendication 1 qui est le (L-acide ascorbique-2-O)(2,5,7,8-tétraméthyl-2-(4,8,12,16-tétraméthylheptadécyl)-chromanyl-6)ester de l'acide phosphorique et un de ses sels.

6. Composé de la revendication 1 qui est le (L-acide ascorbique-2-O)(2,5,7,8-tétraméthyl-2-(4,8,12,16,20-pentaméthylhénéicosyl)chromanyl-6)ester et un de ses sels.

7. Procédé de préparation d'un composé selon les revendications 1-6, comprenant la séquence suivante de ; traitement d'un dérivé de chromane choisi dans le groupe de formule

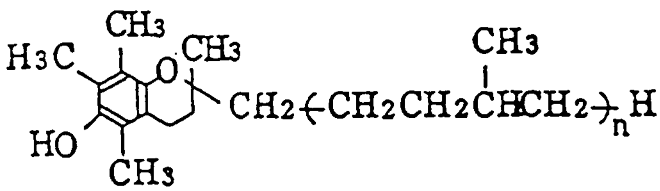

2

avec un agent halogénophosphorylant,puis soumission du phosphodihalogénure de chromanyle résultant à la réaction avec l'acide ascorbique dont les groupes hydroxy en $C_5$ et $C_6$ sont protégés avec un ou plusieurs groupes protecteurs, et élimination du ou des groupes protecteurs du produit de la réaction.

8. Procédé de la revendication 7, dans lequel l'agent halophosphorylant est l'oxychlorure de phosphore ou l'oxybromure de phosphore.

9. Procédé de la revendication 7, dans lequel le traitement du dérivé de chromane avec l'agent halogénophosphorylant est conduit dans le benzène, le toluène ou le xylène en présence de pyridine ou de triéthylamine.

10. Procédé de la revendication 7, dans lequel le groupe protecteur est l'isopropylidène ou l'acétyle.

11. Procédé de la revendication 7, dans lequel la réaction du phosphodihalogénure de chromanyle avec un acide ascorbique dont les groupes hydroxy en $C_5$ et $C_6$ sont protégés par un ou plusieurs groupes protecteurs est conduite dans le tétrahydrofuranne en présence de pyridine ou de triéthylamine.

12. Procédé de la revendication 7, dans lequel l'élimination du ou des groupes protecteurs se fait dans des conditions acides en employant l'acide chlorhydrique, l'acide sulfurique ou l'acide phosphorique.

13. Utilisation des composés selon les revendications 1 à 6, ou obtenus par le procédé des revendications 7 à 12, comme antioxydants.

14. Composition comprenant un composé selon les revendications 1 à 6, ou obtenu par le procédé des revendications 7 à 12, comme antioxydant.

15. Composition de la revendication 14 pour utilisation dans les aliments traités et les cosmétiques aqueux.

**Revendication pour l'Etat conctractant suivant : ES**

1. Procédé de préparation d'un composé choisi dans le groupe de formule :

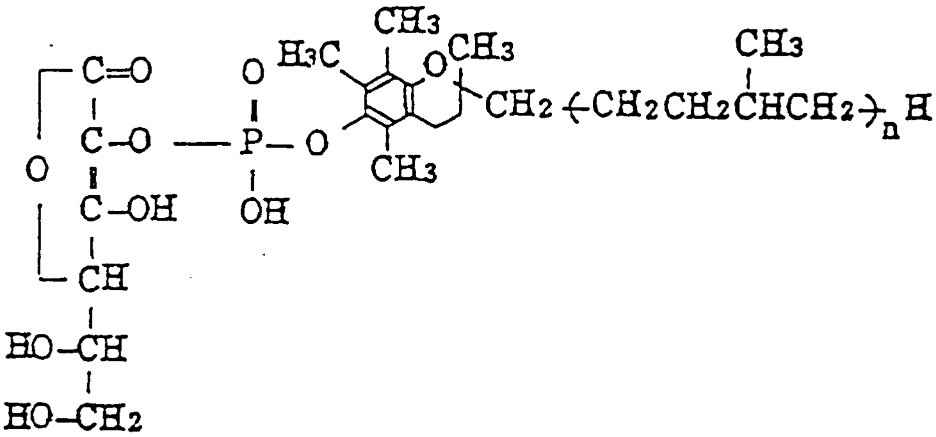

dans laquelle n est un nombre entier choisi dans le groupe constitué par 0, 1, 2, 4 et 5 et un de ses sels qui comprend la séquence suivante de : traitement d'un dérivé de chromane choisi dans le groupe de formule :

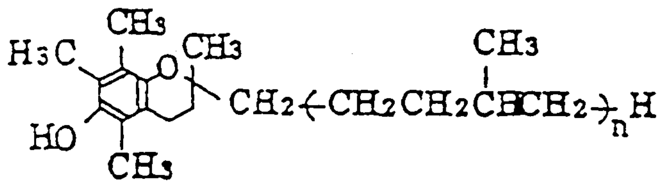

avec un agent halogénophosphorylant, puis soumission du phosphodihalogénure de chromanyle résultant à la réaction avec un acide ascorbique dont les groupes hydroxy en $C_5$ et $C_6$ sont protégés avec un ou plusieurs groupes protecteurs, et élimination du ou des groupes protecteurs du produit de la réaction.

2. Procédé de la revendication 1, dans lequel le produit final est (L-acide ascorbique-2-O)(2,5,7,8-tétraméthyl-2-(4,8-diméthylnonyl)chromanyl-6)-ester de l'acide phosphorique ou un de ses sels.

3. Procédé de la revendication 1, dans lequel le produit final est (L-acide ascorbique-2-O)(2,2,5,7,8-pentaméthyl-chromanyl-6)ester de l'acide phosphorique, ou un de ses sels.

4. Procédé de la revendication 1, dans lequel le produit final est (L-acide ascorbique-2-O)(2,5,7,8-tétraméthyl-2-(4-méthylpentyl)chromanyl-6)ester de l'acide phosphorique ou un de ses sels.

5. Procédé de la revendication 1, dans lequel le produit final est (L-acide ascorbique-2-O)(2,5,7,8-tétraméthyl-2-(4,8,12,16-tétraméthylheptadécyl)chromanyl-6)ester de l'acide phosphorique ou un de ses sels.

6. Procédé de la revendication 1, dans lequel le produit final est (L-acide ascorbique-2-O)(2,5,7,8-tétraméthyl-2-(4,8,12,16,20-pentaméthylhénéicosyl)chromanyl-6)ester de l'acide phosphorique ou un de ses sels.

7. Procédé de la revendication 1, dans lequel l'agent halogénophosphorylant est l'oxychlorure de phosphore ou l'oxybromure de phosphore.

8. Procédé de la revendication 1, dans lequel le traitement du dérivé de chromane avec l'agent halogénophosphorylant se fait dans le benzène, le toluène ou le xylène en présence de pyridine ou de triéthylamine.

9. Procédé de la revendication 1, dans lequel le groupe protecteur est l'isopropylidène ou l'acétyle.

10. Procédé de la revendication 1, dans lequel la réaction de phosphodihalogénure de chromanyle avec un acide ascorbique dont les groupes hydroxy en $C_5$ et $C_6$ sont contenus par un ou plusieurs groupes protecteurs se fait dans le tétrahydrofuranne en présence de pyridine ou de triéthylamine.

11. Procédé de la revendication 1, dans lequel l'élimination du ou des groupes protecteurs se fait dans des conditions acides en employant l'acide chlorhydrique, l'acide sulfurique, ou l'acide phosphorique.

12. Composition comprenant un composé obtenu par le procédé des revendications 1 à 11 comme antioxydant.

13. Aliment traité comprenant un composé obtenu par les revendications 1 à 11.

14. Cosmétique aqueux comprenant un composé obtenu par les revendications 1 à 11.